# EUROPEAN PATENT APPLICATION

(11) **EP 3 009 514 A1**
(43) Date of publication of application: **20.04.2016**
(21) Application number: 14306619.9
(22) Date of filing: 14.10.2014
(51) Int. Cl.: C12N 15/87, C08K 3/34

(54) **Method for transfecting eukaryotic cells using sepiolite fibres**

(71) Applicant: Institut Gustave Roussy, 94805 Villejuif Cédex (FR)
(72) Inventor: Castro Smirnov, Fidel Antonio, 10600 La Habana (CU); Lopez, Bernard, 91190 Gif Sur Yvette (FR); Ruiz Hitzky, Eduardo, 28027 Madrid (ES); Aranda Gallego, Pilar, 28027 Madrid (ES)
(74) Representative: Casalonga

(57) **Abstract**

The present invention relates to a method *for in vitro* or *ex vivo* transfecting eukaryotic cells with one or more nucleic acids wherein said cells are contacted with a transfection composition containing one or more composite materials comprising sepiolite fibres, having a mean length being less than or equal to 2µm, linked with said nucleic acids.

The invention further concerns a pharmaceutical composition containing such composite material, and the composite material for its use for transfecting mammalian cells.

## Description

### FIELD OF THE INVENTION

The present invention relates to a method for *in vitro* or *ex vivo* transfecting eukaryotic cells with one or more nucleic acids, using a transfection composition containing one or more composite materials comprising sepiolite fibres.

The present invention further relates to a composite material comprising sepiolite fibres for its use for transfecting mammalian cells, and in particular human cells.

### BACKGROUND AND GENERAL DESCRIPTION OF THE

### INVENTION

Transfection refers to a process which consists in deliberately introducing a biological functional nucleic acid into the nucleus of a eukaryotic cell. The foreign nucleic acid is then expressed and retains its functions within the cell.

This process leads either to a stable transfection, when the foreign nucleic acid is integrated in the genome of the cell, or to a transient transfection, when the foreign nucleic acid is expressed for a limited time.

The transfer of particular nucleic acids into biological organisms is a pivotal issue to design novel promising strategies for gene therapy and/or the development of new biological models of interest for both academic and applied biological/biotechnological, medical and agronomy research.

A broad range of non-viral delivery systems has been developed leading to both chemical and physical transfection methods.

Common physical transfection methods include electroporation, hydroporation, sonoporation, biolistic technology, micro/nanoinjection and laserfection/optoinjection. These methods usually employ a physical force to overcome the membrane barrier of the cells and thus facilitate the intracellular transfer of nucleic acids. The application of these methods is quite simple, since the nucleic acid is introduced into the cell without involving a particular carrier or a viral system. However, these methods require expensive equipments. They are also time-demanding and induce several drawbacks, including cell mortality and tissue damages. Furthermore, prospects for *in vivo* delivery are poor.

Chemical transfection methods typically rely on the use of electrostatic interactions between cationic compounds and the negatively charged backbone of nucleic acids. Thus, the transfection reagents are commonly composed of cationic liposomes and cationic polymers, which form electrostatic complexes with the nucleic acids. Although these methods require no particular equipment, their reactants remain expensive and their applications are not fully satisfactory. Indeed, these methods comprise generally several steps that can be difficult to control, and their efficiency depends on the cell type. In addition, they are time-demanding, difficult to reproduce and can become toxic at high concentrations.

Thus, there is a real need to provide a non-viral transfection method that overcomes the drawbacks previously mentioned. The method shall be inexpensive, easy to reproduce and shall induce a high transfection efficiency.

The method shall also trigger both stable and transient transfection and shall be suitable for eukaryotic cell types.

The Applicant has now discovered, surprisingly, that an efficient transfection of eukaryotic cells could be achieved using a composite material comprising particular inorganic fibres that are linked with the nucleic acids to be transfected into the cell.

The present invention therefore relates to a method for *in vitro* or *ex vivo* transfecting eukaryotic cells with one or more nucleic acids wherein said cells are contacted with a transfection composition containing one or more composite materials comprising sepiolite fibres, having a mean length being less than or equal to 2µm, linked with said nucleic acids.

The method of the invention makes it possible to achieve the objectives outlined above.

The transfection method according to the invention requires no particular expensive equipment.

The Applicant has discovered that the particular sepiolite fibres used in the present invention internalize spontaneously and rapidly into the eukaryotic cells, and in particular into mammalian cells. The transfection method according to the invention is therefore not time-consuming.

Moreover, the Applicant has discovered, surprisingly, that the transfection method according to the invention allows the nuclear internalization of the nucleic acids.

The method according to the invention triggers both stable and transient transfection of different types of nucleic acids in different types of eukaryotic cells.

Besides, the particular sepiolite fibres used in the invention are considered as being non-hazardous and non carcinogenic by the International Agency of Research on Cancer (IARC) and the European Directive. The method of the invention constitutes a promising approach for gene therapy, in particular for the treatment of genetic disorders, cardiovascular diseases, AIDS and cancer.

A first subject of the present invention is thus a non therapeutic method for *in vitro* or *ex vivo* transfecting eukaryotic cells with one or more nucleic acids wherein said cells are contacted with a transfection composition containing one or more composite materials comprising sepiolite fibres, having a mean length being less than or equal to 2µm, linked with said nucleic acids.

Similarly, the present invention also relates to a composite material comprising sepiolite fibres, having a mean length being less than or equal to 2µm, linked with one or more nucleic acids, for its use for transfecting mammalian cells.

Other subjects, characteristics, aspects and advantages of the invention will emerge even more clearly on reading the description and the examples that follow.

In which that follows and unless otherwise indicated, the limits of a range of values are included within this range, in particular in the expressions "of between" and "ranging from...to...".

Moreover, the expression "at least one" used in the present description is equivalent to the expression "one or more".

### FIGURES

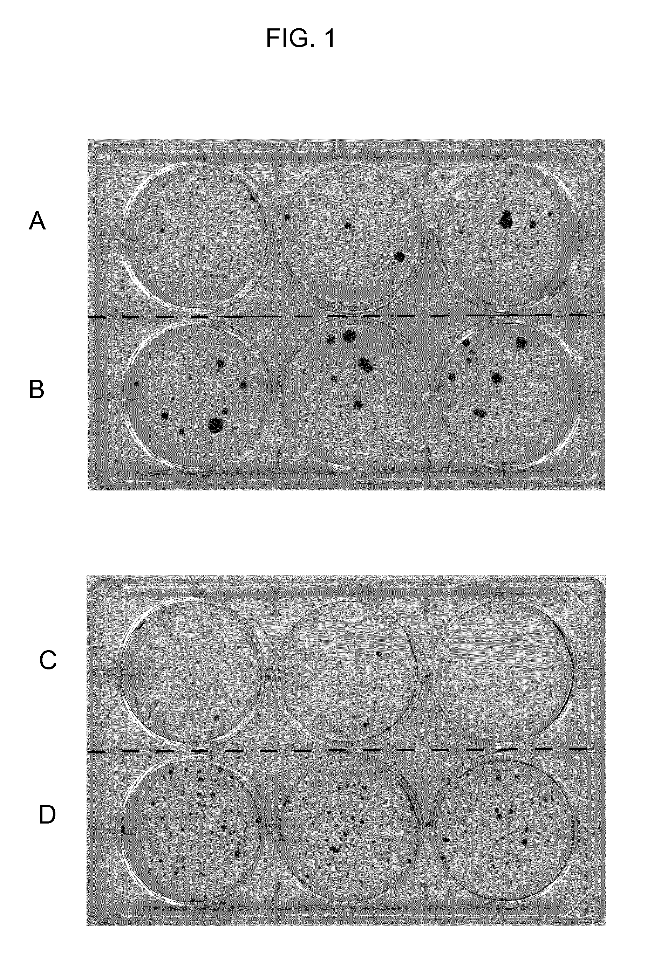

Figure 1 shows the efficiency of the transfection method of the present invention on V79 hamster cells (1A and 1B) and on U2OS human cells (1C and 1D). V79 cells and U2OS human cells were transfected with composite materials comprising either sepiolite fibres (1A and 1C) or sonicated sepiolite fibres (1B and 1D) linked with the gene resistant to G418, in presence of CaCl₂.

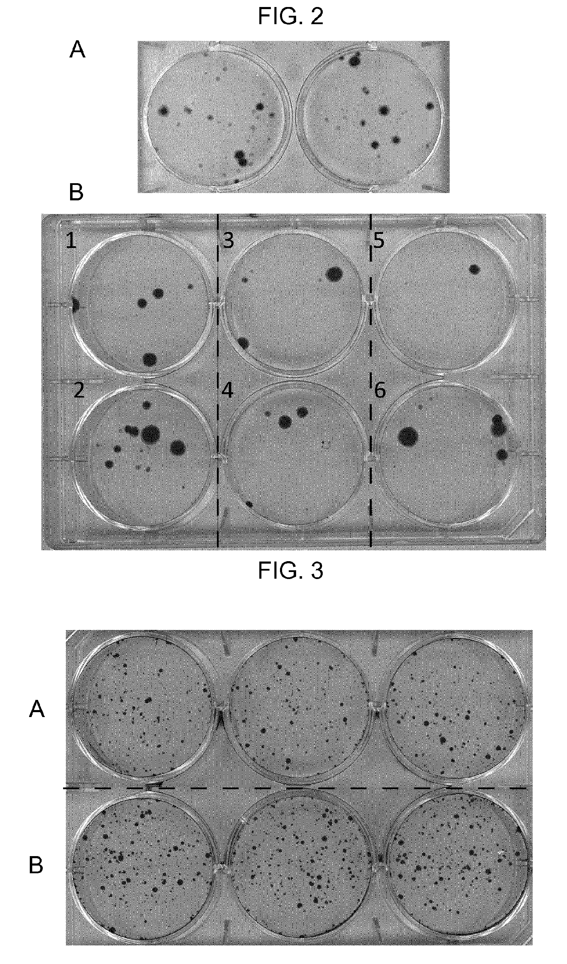

Figure 2 shows the efficiency of the transfection method of the present invention in presence of different polyvalent cations, Mg²⁺ (2A, left), Ca²⁺ (2A, right and 2B, 1-2), spermidine (2B, 3-4) and spermine (2B, 5-6), on V79 hamster cells.
Figure 3 shows the efficiency of the transfection method of the present invention using sonicated sepiolite fibres on U2OS human cells. U2OS cells pretreated with chloroquine (3B) were compared to untreated cells (3A). Both types of cells were then transfected with composite materials comprising sonicated sepiolite fibres.

### DETAILED DESCRIPTION OF THE INVENTION

The present invention is based on the use of particular composite materials that comprise sepiolite fibres, having a mean length being less than or equal to 2µm.

Sepiolite is a natural hydrated magnesium silicate of theoretical unit cell formula Si₁₂O₃₀Mg₈(OH,F)₄(H₂O)₄,8H₂O. Sepiolite shows an alternation of blocks and tunnels that grow up in the fibre direction. The blocks are constituted by two layers of tetrahedral silica sandwiching a central magnesium oxide-hydroxide layer. The discontinuity of the silica sheets gives rise to the presence of silanol groups (Si-OH) at the edges of the channels, which are the tunnels open to the external surface of the sepiolite particles.

According to a preferred embodiment, the total specific surface area of the sepiolite fibres, determined by BET measurements, ranges from 100 to 500 m²/g, preferably from 200 to 400 m²/g.

In a preferred embodiment, the mean length of the sepiolite fibres is less than or equal to 1µm, and better still less than or equal to 800nm. More preferably, the mean length of the sepiolite fibres ranges from 150 to 850nm.

The sepiolite used in the composite material of the invention contains advantageously less than 1% by weight of fibres having a length of at least 5µm, preferably less than 5% by weight of fibres having a length of at least 2.5µm.

The size distribution (length and diameter) of the fibres can be determined by transmission electron microscopy (TEM), using a Zeiss 912AB transmission electron microscope (dark-field mode with a tilted illumination).

In addition, the mean diameter of the sepiolite fibres of the present invention is preferably less than or equal to 50nm, more preferably less than or equal to 25nm, and better still ranges from 8 to 18nm.

Examples of sepiolite fibres that can be used in the present invention are the products sold by the company Tolsa S.A., under the name Pangel S9, and by the company Kremer Pigmente GmbH&Co, under the number 58945.

According to a particular preferred embodiment, the sepiolites fibres are sonicated prior to the preparation of the composite material, that is to say before contacting them with the nucleic acids.

Indeed, the Applicant has discovered that sonicating the sepiolite fibres increases the affinity between the fibres and the nucleic acids, and improves the overall efficiency of the transfection.

The concentration of the sepiolite fibres in the transfection composition of the present invention typically ranges from 0.1 to 5µg.µl⁻¹, preferably from 0.5 to 4µg.µl⁻¹, and better still from 1 to 3µg.µl⁻¹.

The composite material used in the present invention comprises one or more nucleic acids that are linked to the sepiolite fibres.

When two or more distinct nucleic acid chains are present in the transfection composition, the method of the invention is a method of co-transfection. The term distinct means that the nucleic acid chains have different structures.

In the present invention, the nucleic acids can be in particular chosen from DNA, RNA, DNA/RNA hybrids and chemically modified nucleic acids. More preferably the nucleic acids are chosen from DNA chains and RNA chains.

According to a preferred embodiment, the nucleic acids are chosen from linear or circular, single stranded or double-stranded nucleic acid chains. The nucleic acids can be present in any kind of vector. Preferably, the vector is a plasmid, such as pCMV.

According to another preferred embodiment, the nucleic acids are chosen from single stranded or double stranded RNA chains. Preferably, the double stranded RNA is a siRNA.

The concentration of the nucleic acids in the transfection composition of the present invention can typically range from 0.05 to 0.9µg.µl⁻¹, preferably from 0.08 to 0.75µg.µl⁻¹.

The composite material of the present invention can be prepared by adding the above described nucleic acids to a suspension of the sepiolite fibres in a liquid medium. The nucleic acids spontaneously link to the sepiolite fibres.

The liquid medium is preferably chosen from water, a growth medium or a Tris-HCl buffer.

According to a preferred embodiment, the liquid medium is a 10mM Tris-HCl buffer, with a pH value equals to 7.5.

According to a preferred embodiment, the suspension of sepiolite fibres is sonicated prior to the addition of the nucleic acids.

The transfection composition of the present invention advantageously contains said composite material in a liquid medium as described above.

The transfection composition is typically prepared by adding said nucleic acids directly to the sepiolite fibres in suspension in said liquid medium.

In the composite material of the present invention, the weight ratio between the amount of said nucleic acids and the amount of said sepiolite fibres advantageously ranges from 20 to 300µg of nucleic acids linked per mg of sepiolite fibres, preferably from 50 to 270µg of nucleic acids linked per mg of sepiolite fibres, and better still from 70 to 200µg of nucleic acids linked per mg of sepiolite fibres.

According to a particular embodiment of the invention, the composite material further comprises one or more additional molecules that are also linked to the sepiolite fibres, chosen from biological molecules, chemical molecules and mixtures thereof.

In particular, the biological molecules are chosen from polysaccharides, viruses, lipids, proteins and mixtures thereof, and more preferably from proteins.

The chemical molecules are advantageously chosen among toxins having a therapeutic interest, such as chloroquine.

This embodiment can be achieved by adding said biological or chemical molecules directly into the transfection composition described above.

The transfection composition, according to the present invention, may further contain one or more cations, chosen from monovalent cations, polyvalent cations and mixtures thereof.

Indeed, the Applicant has discovered that in presence of cations the affinity between the sepiolite fibres and the nucleic acids is increased, leading to a significant improvement of the overall efficiency of the transfection.

In a preferred embodiment, the cations which can be present in the transfection composition are polyvalent cations. They are preferably chosen from:
- divalent inorganic cations, such as Mg²⁺ and Ca²⁺, and
- polycationic aliphatic amines, preferably trivalent and tetravalent amines, such as spermidine and spermine.

According to a particularly preferred embodiment, the cations are divalent inorganic cations, and are even more preferably chosen from Mg²⁺, Ca²⁺ and mixtures thereof.

The cations, when they are present in the transfection composition of the present invention, have a concentration preferably ranging from 0.5 to 100mM, and more preferably from 0.5 to 80mM.

The method of the present invention is a method for *in vitro*, *ex vivo* or *in vivo* transfecting eukaryotic cells. According to a preferred embodiment, the eukaryotic cells are mammalian cells. As preferred examples of mammalian cells, one can mention, human, monkey, canine, feline, bovine and murine cells. More preferentially, the mammalian cells are human cells.

The eukaryotic cells are advantageously mammalian cancer cells, and more preferably human cancer cells.

According to another particular embodiment, the eukaryotic cells are insect cells.

The eukaryotic cells of the present invention may come either from established cell lines or from primary cell cultures. Preferably, the eukaryotic cells come from established cell lines. Examples of established cell lines are the Chinese hamster cells (V79 cells) and the human osteosarcoma cells (U2OS cells).

In addition, the eukaryotic cells of the present invention can be either dividing cells or non-dividing cells, preferably dividing cells.

The eukaryotic cells of the invention can be either somatic or stem cells, preferably somatic cells.

The eukaryotic cells are cultured in an appropriate growth medium, which can be supplemented with several growth factors, nutrients and antibiotics. Examples of suitable growth media include modified Eagle's medium (MEM) and Dulbecco's modified Eagle's medium (DMEM), depending on the cell line.

However, the choice of the growth medium will be appreciated by those skilled in the art, because some cells require a special growth medium. A person skilled in the art will therefore adapt the growth medium preparation to the studied cells, including pH, glucose concentration, growth factors, nutrients and antibiotics.

Preferably, the growth medium used in the present invention comprises 10% by volume of fetal bovine serum (FBS), relative to the total volume of the growth medium.

In addition, the culture conditions, such as the temperature and the percentage of CO₂, will be adapted by a person skilled in the art, depending on the cell types. According to a prefer embodiment, the eukaryotic cells are incubated at 37°C and 5% of CO₂.

The optimal density of cells depends on the cell types and on the particular purpose of the transfection method.

The growth medium can further contain one or more additional component. Preferably, the growth medium comprises chloroquine.

Prior to contacting the cells with the transfection composition, the growth medium is preferably removed and replaced by a fresh growth medium. Preferably, the fresh growth medium comprises chloroquine.

According to a preferred embodiment of the invention, the eukaryotic cells are incubated with chloroquine prior to contacting them with the transfection composition.

Indeed, the Applicant has discovered that the transfer of nucleic acids is enhanced when the cells have been previously treated with chloroquine. The overall efficiency of the transfection is therefore improved.

The eukaryotic cells are preincubated with chloroquine, by contacting them with chloroquine, for a period advantageously ranging from 5 to 60 minutes, and more preferably from 15 to 45 minutes before the introduction of the transfection composition.

The chloroquine may be present in a concentration ranging from 2 to 100µM, and preferably from 5 to 25µM with regard to the cell growth medium.

The method for *in vitro* or *ex vivo* transfecting eukaryotic cells according to the present invention comprises a step wherein the eukaryotic cells are contacted with the transfection composition described above.

This can be advantageously achieved by introducing the transfection composition directly into the growth medium containing said cells.

According to another preferred embodiment, the transfection composition is directly contacted with the cells, before adding the growth medium.

The transfection method, according to the present invention, requires no mechanical action, such as a mechanical friction, of the sepiolite fibres on the eukaryotic cell membranes in order to enable the cellular internalisation of the composite materials. The composite material is just contacted with the eukaryotic cells, without any mechanical action on the cells and/or the composite material.

The concentration of the sepiolite fibres in the medium surrounding said eukaryotic cells, after contacting said eukaryotic cells with the transfection composition, ranges preferably from 0.1 to 50 ng.µl⁻¹, more preferably from 1 to 20 ng.µl⁻¹, and better still from 5 to 10 ng.µl⁻¹.

The medium surrounding the eukaryotic cells during the transfection itself is typically made of a mixture of the growth medium and the transfection composition.

According to a preferred embodiment, the eukaryotic cells are contacted with the transfection composition for a period that is superior than or equal to one hour.

More preferably, the eukaryotic cells are contacted with the transfection composition for a period ranging from 1 to 60 hours, and better still from 6 to 48 hours.

After the transfection has been sufficiently completed, the process can be stopped simply by replacing the liquid medium surrounding the cells, which is typically made of a mixture of the growth medium and of the transfection composition, with a fresh growth medium that does not contain the said composite material.

The efficiency of the transfection method can be analyzed by different methods. The target gene expression level can be detected by reporter genes, such as for example mKate2 gene, luciferase gene, or β-galactosidase gene expression. The signal of mKate2 can be directly observed under a fluorescence microscope, the activity of luciferase can be detected by a luminometer, and the blue product catalyzed by β-galactosidase can be observed under microscope or determined by a microplate reader. A person skilled in the art is familiar with how these reporters function and how they may be introduced into a gene delivery system.

The nucleic acid and its expression products can be determined by various methods, such as detecting immunofluorescence or enzyme immunocytochemistry, autoradiography or in situ hybridization. If immunofluorescence is used to detect expression of an encoded protein, a fluorescently labelled antibody that binds the target protein is used. Cells containing the protein are then identified by detecting a fluorescent signal. If the delivered molecules can modulate gene expression, the target gene expression level can also be determined by methods such as autoradiography, in situ hybridization, and in situ PCR.

Another useful method for assessing the efficiency of the transfection, that was used in the examples hereunder, consists in transfecting the cells with a plasmid encoding for a gene resistant to a particular antibiotic, such as G418. The transfected cells become then resistant to the antibiotic and can therefore be easily selected by simply adding the antibiotic to the medium containing the cells. The number of transfected cells can then be visually estimated.

The identification method can be chosen depending on the properties of the delivered nucleic acids and/or their expression products.

Another object of the present invention is a pharmaceutical composition containing, in a pharmaceutically acceptable carrier, one or more composite material(s) as described above. Such material comprises sepiolite fibres, having a mean length being less than or equal to 2µm, linked with one or more nucleic acids.

In a preferred embodiment, the pharmaceutical composition further contains one or more polyvalent cations, preferably chosen from:
- divalent inorganic cations, such as Mg²⁺ and Ca²⁺, and
- polycationic aliphatic amines, preferably trivalent and tetravalent amines, such as spermine and spermidine.

Another object of the present invention is a composite material as described above, for its use for transfecting mammalian cells. Such material comprises sepiolite fibres, having a mean length being less than or equal to 2µm, linked with one or more nucleic acids.

In a preferred embodiment, the composite material of the present invention is for use in gene therapy.

More preferably, the composite material of the present invention is for use in the treatment of cancer, cardiovascular diseases, AIDS, genetic disorders or autoimmune disorders, and better still in the treatment of cancer.

The cancer may be any kind of cancer or tumour, including a carcinoma, a sarcoma, a lymphoma, a melanoma, a paediatric tumour and a leukaemia tumour.

In particular, the cancer may be any kind of cancer including a gastrointestinal sarcoma, a renal cancer, a breast cancer, a leukaemia, an Hodgkin lymphoma, a neuroblastoma, a prostate cancer, an oesophagus cancer, a colon cancer, a rectal cancer, an ovarian cancer, a pancreatic cancer, a testicular cancer, a bladder cancer, a lung cancer, a thyroid cancer, an osteosarcoma and a melanoma.

According to another preferred embodiment, the composite material of the present invention is for use in the immunization of mammals, and more preferably in the immunization of humans.

Other characteristics and advantages of the invention are given in the following experimental section, which should be regarded as illustrative and not limiting the scope of the present application.

### EXAMPLES

### Sepiolite suspensions

Two sepiolite suspensions were prepared by dispersing sepiolite fibres (Pangel S9 from Tolsa, having a mean length ranging from 200 to 600nm,and a mean diameter of 15nm) in a Tris-HCl buffer (10mM, pH=7.5) to obtain a final concentration of sepiolite fibres of 2µg.µl⁻¹. The first sepiolite suspension (Sep) was vigorously mixed for at least 10 minutes, while the second sepiolite suspension (sSep) was sonicated three times at 30% of amplitude for 10 seconds each time with a Vibra cell 75042 (Bioblock Scientific).

### Cell cultures

Chinese hamster cells (V79 cells) were grown in dishes as monolayers in modified Eagle's medium (MEM) supplemented with 10% by volume of fetal bovine serum (FBS), relative to the total volume of the medium.

Human osteosarcoma cells (U2OS cells) were grown in dishes as monolayers in Dulbecco's modified Eagle's medium (DMEM) supplemented with 10% by volume of FBS, relative to the total volume of the medium.

The V79 and the U2OS cells were incubated at 37°C, with 5% CO₂ in air and 100% humidification.

MEM, DMEM and FBS were purchased from Life Technologies^{™}.

V79 and U2OS cells were split in 6 well/plates at a density of 2*10⁴ and 1*10⁴ respectively.

### Example 1

(a) For transfecting V79 cells, 70µl of a transfection composition were prepared by mixing:
   - 40 µl of sepiolite suspension (Sep or sSep) as described above,
   - 14 µl of 50 mM CaCl₂,
   - 7.8 µl of 10 mM TrisHCl and
   - 8.2 µl of pCMV (0.49µg.µl⁻¹) encoding for the gene resistant to G418.

To compare the transfection efficiency in presence of non sonicated and sonicated sepiolite fibres in V79 cells, two transfection compositions were prepared: one using the non sonicated suspension of sepiolite fibres (Sep) and the other using the sonicated suspension of sepiolite fibres (sSep).

After 2 hours stirring, 5.83ml of MEM, supplemented with FBS as described above, were added, and two transfection compositions were thus obtained.
(b) For transfecting U2OS cells, 70µl of a transfection composition were prepared by mixing:
   - 40 µl of sepiolite suspension (Sep or sSep) as described above,
   - 14 µl of 50 mM CaCl₂, and
   - 16 µl of pCMV (0.49 µg.µl⁻¹) encoding for the gene resistant to G418.

To compare the transfection efficiency in presence of non sonicated and sonicated sepiolite fibres in U2OS cells, two transfection compositions were prepared: one using the non sonicated suspension of sepiolite fibres (Sep) and the other using the sonicated suspension of sepiolite fibres (sSep).

After 2 hours stirring, 8.9ml of DMEM, supplemented with FBS as described above, were added, and a transfection composition was thus obtained.

The growth medium of both V79 and U2OS cells was then removed and replaced by 3ml of respectively each transfection compositions (a) and (b) as described above.

After two days, the cells were washed with PBS and fresh growth medium, supplemented with antibiotic G418 (600 ng.µl⁻¹ for V79 cells and 0.8 µg.µl⁻¹ for U2OS cells), was added, in order to start the selection.

10 days later, the cells colonies were stained with Giemsa (25% in ethanol) and the number of colonies was estimated.

The more colonies there are, the better the transfection. The results obtained are shown in Figure 1.

Figure 1 shows that a satisfying transfection is achieved in both cases and for both cell types. The transfection is further increased when the sepiolite fibres, used in the composite material, were sonicated. Indeed, the cells transfected using sonicated sepiolite fibres (Figures 1B and 1D) show an increased resistance to the antibiotics with regard to the cells transfected using non sonicated sepiolite fibres (Figures 1A and 1C).

### Example 2

70µl of a transfection composition were prepared by mixing:
- 40 µl of non sonicated sepiolite suspension (Sep) as described above,
- 14 µl of 50 mM CaCl₂,
- 7.8 µl of 10 mM TrisHCl and
- 8.2 µl of pCMV (0.49µg.µl⁻¹) encoding for the gene resistant to G418.

To compare the transfection efficiency in presence of different polyvalent cations, four transfection compositions were prepared, using the mixture as described above in which CaCl₂ was replaced with MgCl₂ (50 mM), spermine (1 mM) and spermidine (1 mM).

The mixtures were then stirred at 25°C and 700rpm in a Thermomixer (Eppendorf).

After 2 hours, 5.83ml of MEM, supplemented with FBS as described above, were added, and four transfection compositions were thus obtained.

The growth medium of the V79 cells was then removed and replaced by 3ml of each transfection composition.

After two days, the cells were washed with PBS and fresh growth medium, supplemented with antibiotic G418 (0.6µg.µl⁻¹), was added, in order to start the selection.

10 days later, the cells colonies were stained with Giemsa (25% in ethanol) and the number of colonies was estimated.

The more colonies there are, the better the transfection. The results obtained are shown in Figure 2.

Figure 2 shows that the presence of divalent cations improves the transfection efficiency. furthermore, the cells transfected in presence of either Mg²⁺ (Figure 2A, left) or Ca²⁺ (Figure 2A, right and Figure 2B, 1-2) present more resistance to the antibiotics than the cells transfected in the presence of either spermidine (Figure 2B, 3-4) or spemine (Figure 2B, 5-6).

### Example 3

70µl of a transfection composition were prepared by mixing:
- 40 µl of sonicated sepiolite suspension (sSep) as described above,
- 14 µl of 50 mM CaCl₂, and
- 16 µl of pCMV (0.49µg.µl⁻¹) encoding for the gene resistant to G418.

After 2 hours stirring, 8.9ml of DMEM, supplemented with FBS as described above, were added, and a transfection composition was thus obtained.

The transfection was performed on U2OS cells as described above.

The transfection was also performed on U2OS cells that were pretreated with chloroquine, by incubating them 30 minutes in fresh growth medium supplemented with chloroquine (10µM) prior to the transfection.

In each case, the cell growth medium was removed and replaced by 3ml of the transfection composition to start the transfection.

After two days, the cells were washed with PBS and fresh growth medium, supplemented with antibiotic G418 (0.8µg.µl⁻¹), was added, in order to start the selection.

10 days later, the cells colonies were stained with Giemsa (25% in ethanol) and the number of colonies was estimated.

The more colonies there are, the better the transfection. The results obtained are shown in Figure 3.

Figure 3 shows that the transfection efficiency is increased when the cells are pretreated with chloroquine. Indeed, cells treated with chloroquine and then transfected with sonicated sepiolites fibres (Figure 3B) were more resistant to the antibiotics than cells only transfected with sonicated sepiolites fibres (Figure 3A).

## Claims

1. A method for *in vitro* or *ex vivo* transfecting eukaryotic cells with one or more nucleic acids wherein said cells are contacted with a transfection composition containing one or more composite materials comprising sepiolite fibres, having a mean length being less than or equal to 2µm, linked with said nucleic acids.

2. The method according to the preceding claim wherein the mean length of the sepiolite fibres is less than or equal to 1µm, preferably less than or equal to 800nm, and more preferably ranges from 150 to 850nm.

3. The method according to any one of the preceding claims wherein the mean diameter of the sepiolite fibres is less than or equal to 50nm, preferably less than or equal to 25nm, and more preferably ranges from 8 to 18nm.

4. The method according to any one of the preceding claims wherein the sepiolite fibres are sonicated prior to the preparation of the composite material.

5. The method according to any one of the preceding claims wherein the nucleic acids are chosen from DNA, RNA, DNA/RNA hybrids and chemically modified nucleic acids.

6. The method according to the preceding claim wherein the nucleic acids are chosen from linear or circular, single stranded or double-stranded nucleic acids chains.

7. The method according to any one of the preceding claims wherein the weight ratio between the amount of said nucleic acids and the amount of said sepiolite fibres in said composite material ranges from 20 to 300µg of nucleic acids linked per mg of sepiolite fibres, preferably from 50 to 270µg of nucleic acids linked per mg of sepiolite fibres, and better still from 70 to 200µg of nucleic acids linked per mg of sepiolite fibres.

8. The method according to any one of the preceding claims wherein said composite material further comprises one or more additional molecules chosen from biological molecules, chemical molecules and mixtures thereof.

9. The method according to the preceding claim wherein the biological molecules are chosen from polysaccharides, viruses, lipids, proteins and mixtures thereof, and preferably from proteins.

10. The method according to any one of the preceding claims wherein the transfection composition further contains one or more cations, chosen from monovalent cations, polyvalent cations and mixtures thereof.

11. The method according to the preceding claim wherein the cations are polyvalent cations, chosen from:
- divalent inorganic cations, such as Ca²⁺ and Mg²⁺, and
- polycationic aliphatic amines, preferably trivalent and tetravalent amines, such as spermidine and spermine.

12. The method according to any one of the preceding claims wherein said eukaryotic cells come from established cell lines or from primary cell culture.

13. The method according to any one of the preceding claims wherein the said eukaryotic cells are dividing cells or non-dividing cells.

14. The method according to any one of the preceding claims wherein the said eukaryotic cells are insect cells.

15. The method according to any one of the claims 1 to 13 wherein the said eukaryotic cells are mammalian cells, preferably human cells.

16. The method according to the preceding claim wherein said eukaryotic cells are mammalian cancer cells, preferably human cancer cells.

17. The method according to any one of the preceding claims wherein said eukaryotic cells are treated with chloroquine prior to contacting them with the transfection composition.

18. The method according to any one of the preceding claims wherein the concentration of the sepiolite fibres in the medium surrounding said eukaryotic cells, after contacting said cells with the transfection composition, ranges from 0.1 to 50 ng.µl⁻¹, preferably from 1 to 20 ng.µl⁻¹, and better still from 5 to 10 ng.µl⁻¹.

19. The method according to any one of the preceding claims wherein said eukaryotic cells are contacted with said transfection composition for a period of time superior or equal to 1 hour, preferably for a period ranging from 1 to 60 hours, and better still from 6 to 48 hours.

20. Pharmaceutical composition containing, in a pharmaceutically acceptable carrier, one or more composite material(s) as defined in any one of claims 1 to 9.

21. Pharmaceutical composition according to the preceding claim further containing one or more polyvalent cations as defined in claims 10 or 11.

22. Composite material as defined in any one of claims 1 to 9 for its use for transfecting mammalian cells.

23. Composite material according to the preceding claim for its use in gene therapy.

24. Composite material according to claims 22 or 23 for its use in the treatment of cancer, cardiovascular diseases, AIDS, genetic disorders or autoimmune disorders, and preferably in the treatment of cancer.

25. Composite material according to claim 22 for its use in the immunization of mammals, and preferably in the immunization of humans.
